# EUROPEAN PATENT SPECIFICATION

(11) **EP 4 285 959 B1**
(45) Date of publication and mention of the grant of the patent: **13.05.2026**
(21) Application number: 22176651.2
(22) Date of filing: 01.06.2022
(51) Int. Cl.: A61M 5/142, A61M 5/178

(54) **BATTERY-FREE SENSING SOLUTION FOR DRUG DELIVERY DEVICES**
BATTERIEFREIE SENSORLÖSUNG FÜR MEDIKAMENTENVERABREICHUNGSVORRICHTUNGEN
SOLUTION DE DÉTECTION SANS BATTERIE POUR DISPOSITIFS D'ADMINISTRATION DE MÉDICAMENTS

(43) Date of publication of application: 06.12.2023
(73) Proprietor: Becton, Dickinson and Company, Franklin Lakes, NJ 07417-1880 (US)
(72) Inventor: ALBENGE, Olivier, 38130 Echirolles (FR)
(74) Representative: Germain Maureau

(56) References cited:
- WO-A1-2013/116873
- US-A1- 2009 318 876
- US-A1- 2015 045 727
- US-A1- 2021 085 879

## Description

### BACKGROUND OF THE INVENTION

### Field of the Invention

The present invention relates generally to drug delivery devices and components therefor, and in particular, to components allowing for detection and communication of the injection status of drug delivery devices.

### Description of Related Art

Drug delivery devices have traditionally lacked features that would allow a healthcare provider to confirm appropriate administration of medicaments. Ensuring timely, appropriate administration of some medicaments can be critical, particularly, for example, in the instance of the evaluation of new drugs in clinical trials, where accurate information is essential.

While some devices provide the ability to communicate certain types of information, for example as described in International Patent Application Publication Nos. WO 2013/116873, WO 2016/087512, WO 2017/070391, WO 2018/111969, WO 2018/213837, and WO 2021/094797, and in U.S. Patent Application Publication Nos. 2021/085879, 2019/0083708, 2019/0321555, and 2019/0344019, a need exists in the art for a cost-effective device that accurately detects injection status, and can communicate this drug delivery information to appropriate stakeholders.

### SUMMARY OF THE INVENTION

Provided herein is a drug delivery device including a syringe barrel having a proximal end, a distal end, and a sidewall arranged between the proximal end and the distal end defining an interior configured to hold a composition, a plunger rod received at least partially within the interior of the syringe, the plunger rod having a proximal end, a distal end, and a stopper arranged at the distal end, and a sensor assembly, including a capacitive element and a short-range communication device in electrical communication with the capacitive element, wherein the short-range communication device is capable of measuring capacitance at the capacitive element, and where the drug delivery device is as further defined in claim 1.

Also provided herein is a system including a drug delivery device including a syringe barrel having a proximal end, a distal end, and a sidewall arranged between the proximal end and the distal end defining an interior configured to hold a composition, a plunger rod received at least partially within the interior of the syringe, the plunger rod having a proximal end, a distal end, and a stopper arranged at the distal end, and a sensor assembly, including a capacitive element and a short-range communication device in electrical communication with the capacitive element, wherein the short-range communication device is capable of measuring capacitance at the capacitive element, and where the drug delivery device is as further defined in claim 1. The system also includes an NFC-enabled mobile device.

Also provided herein is a method of monitoring drug delivery status, including steps of providing a drug delivery device including a syringe barrel having a proximal end, a distal end, a needle arranged at the distal end, and a sidewall arranged between the proximal end and the distal end defining an interior configured to hold a composition, a plunger rod received at least partially within the interior of the syringe, the plunger rod having a proximal end, a distal end, and a stopper arranged at the distal end, and a sensor assembly, including a capacitive element and a short-range communication device in electrical communication with the capacitive element, wherein the short-range communication device is capable of measuring capacitance at the capacitive element, and where the drug delivery device is as further defined in claim 1, powering, with a mobile device, the sensor assembly to obtain first capacitance data, and transmitting, with the short-range communication device and to the mobile device, the first capacitance data.

### BRIEF DESCRIPTION OF THE DRAWINGS

**FIGS. 1A** **and** **1B** are perspective and cross-sectional views of a drug delivery device according to non-limiting embodiments described herein;
**FIGS. 2A** **and** **2B** are perspective and cross-sectional views of a drug delivery device according to non-limiting embodiments described herein;
**FIGS. 3A and 3B** are side views of a drug delivery device according to non-limiting embodiments described herein;
**FIG. 4** is a schematic diagram of a system according to non-limiting embodiments described herein;
**FIG. 5** is a diagram of a non-limiting embodiment of an environment in which systems, devices, and/or methods described herein may be implemented; and
**FIG. 6** is a diagram of a non-limiting embodiment of components of one or more devices of **FIG. 5****.**

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

It should be understood that any numerical range recited herein is intended to include all values and sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

As used herein, the terms "communication" and "communicate" refer to the receipt or transfer of one or more signals, messages, commands, or other type of data. For one unit (e.g., any device, system, or component thereof) to be in communication with another unit means that the one unit is able to directly or indirectly receive data from and/or transmit data to the other unit. This may refer to a direct or indirect connection that is wired and/or wireless in nature. Additionally, two units may be in communication with each other even though the data transmitted may be modified, processed, relayed, and/or routed between the first and second unit. For example, a first unit may be in communication with a second unit even though the first unit passively receives data and does not actively transmit data to the second unit. As another example, a first unit may be in communication with a second unit if an intermediary unit processes data from one unit and transmits processed data to the second unit. It will be appreciated that numerous other arrangements are possible. Any known electronic communication protocols, including proprietary communication protocols, and/or algorithms can be used such as, for example, TCP/IP (including HTTP and other protocols), WLAN (including 502.11a/b/g/n and other radio frequency-based protocols and methods), analog transmissions, Global System for Mobile Communications (GSM), UltraWideBand (UWB), 3G/4G/LTE, BLUETOOTH, ZigBee, EnOcean, TransferJet, Wireless USB, and the like known to those of skill in the art. In some non-limiting embodiments, a message may refer to a network packet (e.g., a data packet and/or the like) that includes data.

As used herein, the term "computing device" may refer to one or more electronic devices configured to process data. A computing device may, in some examples, include the necessary components to receive, process, and output data, such as a processor, a display, a memory, an input device, a network interface, and/or the like. A computing device may be a mobile device. As an example, a mobile device may include a cellular phone (e.g., a smartphone or standard cellular phone), a portable computer, a wearable device (e.g., watches, glasses, lenses, clothing, and/or the like), a personal digital assistant (PDA), and/or other like devices. A computing device may also be a desktop computer or other form of non-mobile computer.

As used herein, "electrical communication," refers to a state of communication, for example through a conductive lead, antenna, etc., between one or more capacitive elements, such as a capacitor, and a short-range communication device, such as an NFC or RFID chip/tag, whereby the NFC or RFID chip/tag is capable of detecting and/or measuring a change in capacitance at the capacitive element(s).

Provided herein are drug delivery devices, systems including drug delivery devices, and methods using such systems and devices. Drug delivery devices described herein, as well as systems and methods including the same, include a sensor assembly including a short-range communication device to allow for monitoring of status of the drug delivery device, and communication of that status.

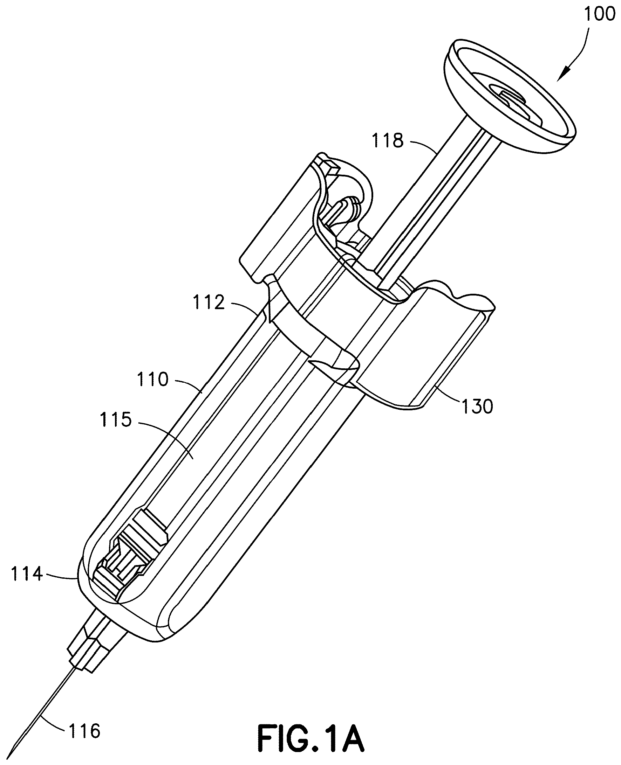

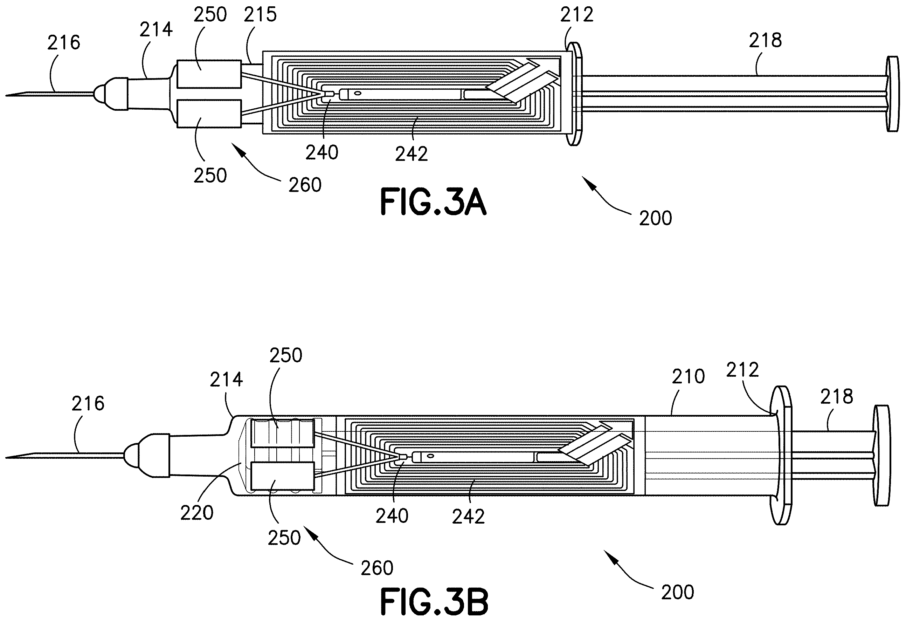

Turning to FIGS. 1A-2B, shown are non-limiting embodiments of a drug delivery device 100. Drug delivery device 100, while shown as a device similar to that disclosed in U.S. Patent No. 10,702,663, may be any drug delivery device, such as a manual syringe (as shown in FIGS. 3A and 3B), an autoinjector, a pen injector, or a wearable pump (e.g., an on-body injector). In the non-limiting embodiment shown in FIGS. 1A-2B, drug delivery device 100 includes a syringe 115 having a proximal end, a distal end, a sidewall therebetween defining an interior configured to hold a composition (e.g., a therapeutic composition) and a needle 116 arranged at the distal end in fluid communication with the interior. Drug delivery device 100 includes a plunger rod 118 having a proximal end (optionally including a thumb pad), a distal end, and a stopper arranged at distal end thereof. Drug delivery device 100 includes a displaceable needle shield 110 having a proximal end 112, a distal end 114, and a spring 120 configured to displace needle shield 110 from a first, proximal position in which needle shield 110 does not surround needle 116 to a second, distal position in which needle shield 110 surrounds needle 116. In the non-limiting embodiment of FIGS. 1A-2B, drug delivery device 100 further includes a clip 130, which may be configured to prevent removal of plunger rod 118 from drug delivery device 100.

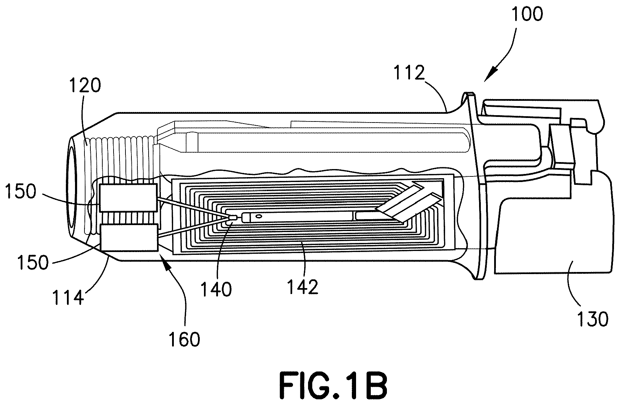

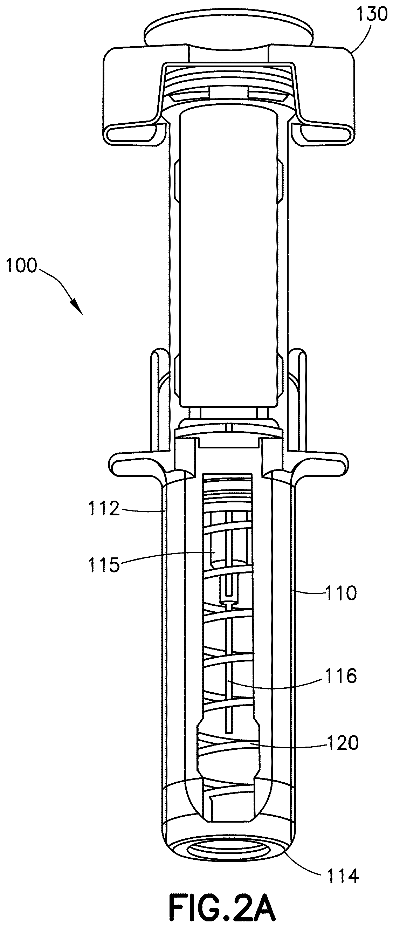

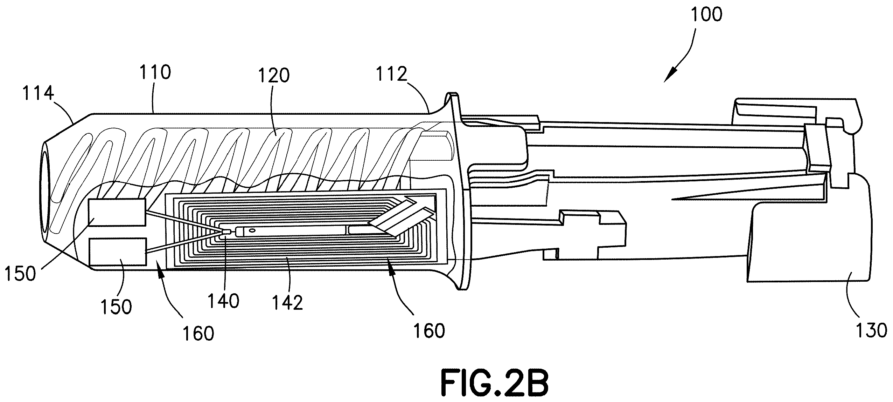

With reference to FIGS. 1B and 2B, drug delivery device 100 is shown in a pre-use (FIG. 1B) and post-use (FIG. 2B) configuration, together with a short-range communication device 140 and one or more capacitive elements 150 (not visible in FIGS. 1A and 2A). Short-range communication device 140 and one or more capacitive elements 150 is considered to be a sensor assembly 160, and may be provided on an internal surface of needle shield 110 or an outer surface of syringe 115, for example as a label. While the accompanying Figures illustrate an exemplary embodiment of sensor assembly 160, those of skill will appreciate that sensor assembly 160 may be arranged on an outer surface of needle shield 110, or embedded within needle shield 110, for example through an in-mold labelling process. This arrangement of sensor assembly 160 allows for determination of the use status of drug delivery device 100 without the need for the sensor assembly 160 to physically contact any other component of the drug delivery device 100 (e.g., no portion of the sensor assembly 160 need be acted upon physically). Sensor assembly 160 may further include one or more processors in communication with short-range communication device 140 and/or memory. In non-limiting embodiments, short-range communication device 140 is a near-field communication (NFC) device. Suitable NFC devices and components therefor are known in the art and are commercially available, for example from NXP Semiconductors (Eindhoven, The Netherlands) and Identiv (Santa Anna, CA). Suitable short-range communication devices may include a chip/tag (optionally including a processor, memory, a cryptographic security processor, and/or one or more additional sensors, such as a temperature sensor), for example for storage of data and/or instructions, and an antenna for communication with NFC-enabled devices, such as computing devices described herein. In non-limiting embodiments, sensor assembly 160 is unpowered (e.g., does not include an internal power source, such as a battery). In non-limiting embodiments, sensor assembly 160 is powered by an external source, for example an NFC-enabled computing device as described herein. As NFC technology allows for powering of the short-range communication device wirelessly, in non-limiting embodiments, data is collected and transmitted without the need to form/break any electrical circuits, and without any contact between drug delivery device 100 and any external device, such as a user's computing device (e.g., a mobile device). The present disclosure also contemplates embodiments where an NFC device is provided inside of a drug delivery device 100, for example, a reusable drug delivery device 100 is contemplated, where an NFC-containing component may be included on or within housing, and a syringe can be inserted/removed from the housing. In non-limiting embodiments, sensor assembly further includes a second communication device, such as an RFID device. In non-limiting embodiments, sensor assembly 160 includes, as short range communication device 140, a dual-frequency NFC chip, which includes multiple antennae, including at least one for NFC and one for RFID communication.

As noted above, sensor assembly 160 includes at least one capacitive element 150. In non-limiting embodiments, at least one capacitive element 150 includes a pair of capacitor plates. Suitable materials for capacitor plates are known to those of skill in the art. Those of skill in the art will also appreciate that the electrical field stored by a capacitor is influenced by the arrangement of the capacitor (e.g., size of and distance between capacitor plates) and the local environment, including materials surrounding and/or adjacent to the capacitor. As the capacitive element 150 is in electrical communication (e.g., via one or more leads) with short-range communication device 140 (and any optional further communication devices, such as an RFID device), short-range communication device (e.g., NFC chip) 140 can, when powered, detect and/or determine capacitance at the capacitive element 150, which, as noted, may be affected by surrounding/adjacent materials. In non-limiting embodiments, sensor assembly 160 is arranged at or near distal end 114 of needle shield 110. In non-limiting embodiments, capacitive element 150 includes a pair of capacitor plates arranged parallel to one another. In non-limiting embodiments, capacitive element 150 includes capacitor plates separated from one another by air or an insulating material, such as ceramic, glass, paper, and/or plastic.

For example, with reference to FIGS. 1A-2B, capacitive element 150 is, in a pre-use position, arranged adjacent to the material (e.g., plastic) of needle shield 110, syringe 115 barrel (e.g., plastic or glass), and any composition received within the interior of syringe 115, as well as spring 120, which in a pre-use position of drug delivery device 100 is compressed (e.g., there is a larger density of spring material (e.g., metal) in the pre-use position than in the post-use position). Further, in the post-use position, capacitive element 150 is adjacent to the material (e.g., plastic) of needle shield 110, syringe 115 barrel (e.g., plastic or glass), (rather than any composition received within the interior of syringe 115), as well as the lower density of spring material as spring 120 is in an expanded configuration. Such a change in environment affects the capacitance of capacitive element 150, which may be detected when power is supplied to sensor assembly, for example by an NFC-enabled computing device associated with a user of drug delivery device 100. Such capacitance data may then, when power is supplied to sensor assembly 160, be stored by short-range communication device 140, and/or communicated, for example through NFC and/or RFID, to a computing device computing device associated with a user of drug delivery device 100. A user may, with an NFC-enabled computing device, provide power to the sensor assembly 160 and obtain capacitance data before and/or after drug delivery.

Turning to FIGS. 3A and 3B, shown is a non-limiting embodiment of a drug delivery device 200 with a short-range communication device 240 and capacitive element 250 (e.g., sensor assembly 260). Drug delivery device 200 in the illustrated embodiment of FIGS. 3A and 3B is a manual syringe, but those of skill will appreciate that the concepts described herein may be applicable to any drug container, such as a pre-filled syringe, for example for use in a pen or autoinjector, pump device, on-body injector, or other drug delivery device. Drug delivery device 200 in the embodiment of FIGS. 3A and 3B includes a syringe barrel 210 having a proximal end 212, a distal end 214, and a sidewall therebetween defining an interior configured to hold a composition. Drug delivery device 200 may include at distal end 214 thereof a needle 216 in fluid communication with interior, and, at proximal end 212, a plunger rod 218 that may include a thumb pad at a proximal end thereof and a stopper 220 at distal end thereof.

As shown in FIGS. 3A and 3B, sensor assembly 260 may be arranged at or near distal end 214 of drug delivery device 200, such that, for example as described above, capacitive element 250 is, in a pre-use position, arranged adjacent to syringe barrel 210 (e.g., plastic or glass), and any composition received within the interior of syringe barrel 210. In the post-use position, capacitive element 250 is adjacent to syringe barrel 210 (e.g., plastic or glass) and the material of the stopper (rather than any composition received within the interior of syringe barrel 210). Such a change in environment affects the capacitance of capacitive element 250, which may be detected when power is supplied to sensor assembly 260, for example by an NFC-enabled computing device associated with a user of drug delivery device 200. Such capacitance data may then, when power is supplied to sensor assembly 260, be stored by short-range communication device 240, and/or communicated, for example through NFC and/or RFID, to a computing device computing device associated with a user of drug delivery device 200. A user may, with an NFC-enabled computing device, provide power to the sensor assembly 260 and obtain capacitance data before and/or after drug delivery.

While the illustrated embodiments (e.g., FIGS. 1A-3B) show an arrangement where sensor assembly 160/260 is arranged such that capacitive element 150/250 is located at or near a distal end 114/214 of drug delivery device 100/200, those of skill will appreciate that sensor assembly 160/260 may alternatively, or in addition, be arranged such that capacitive element 150/250 is located at or near proximal end 112/212 of drug delivery device 100/200. Those of skill will appreciate that the difference in capacitance described above as providing indication of the status of drug delivery device 100/200 will be applicable regardless of the arrangement of capacitive element 150/250.

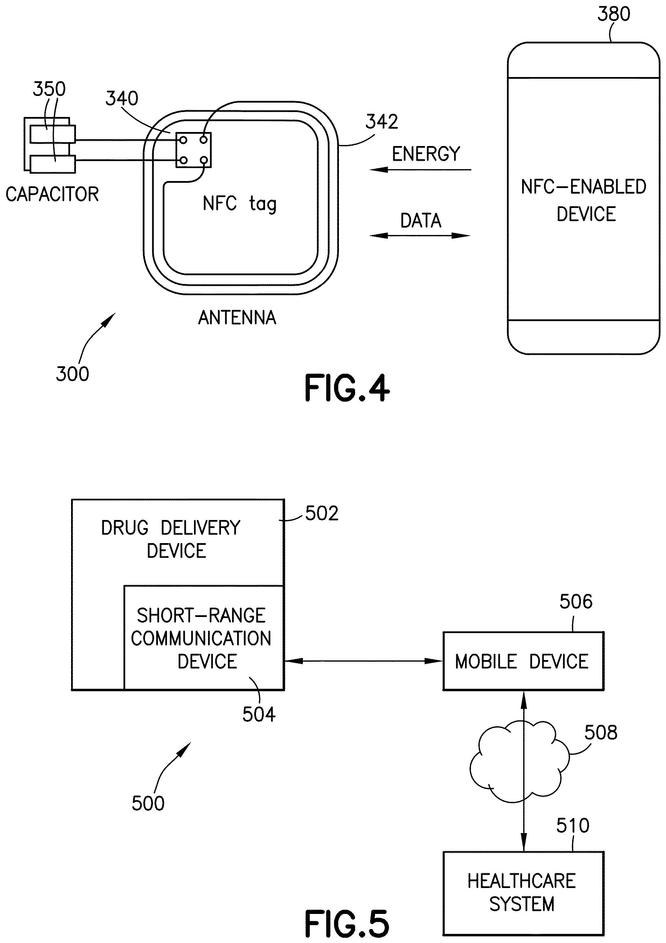

Turning to FIG. 4, shown is an example system 300 environment, including short-range communication device 340, with antenna 342 fully visible due to the close-up perspective, capacitive element 350, and computing device 380 (in the form of a mobile device). Those of skill will appreciate that while not described in detail above, short-range communication devices useful with the aforementioned embodiments may include an antenna. Computing device 380 may be NFC (and, optionally, RFID)-enabled, such that, by bringing computing device 380 close to short-range communication device 340, energy is provided to short-range communication device 340, and capacitance at capacitive element 350 can be detected and/or determined. As capacitance is detected and/or determined, capacitance data detected and/or generated by short-range communication device 340 may be transmitted from short-range communication device 340 to computing device 380.

Referring now to FIG. 5, shown is a diagram of an example environment 500 in which devices, systems, and/or methods, described herein, may be implemented. As shown in FIG. 5, the environment 500 can include drug delivery device 502 including short-range communication device 504, user device 506 (e.g., a user's mobile device), healthcare system 510, and/or communication network 508. Drug delivery device 502, user device 506, and healthcare system 510 may interconnect (e.g., establish a connection to communicate) via wired connections, wireless connections, or a combination of wired and wireless connections.

Drug delivery device 502, which can be a rigid drug container, a syringe, autoinjector, wearable injector, and/or pump as described herein, can include, as described above, a sensor assembly including short-range communication device 504 and capacitive element(s). As described above, drug delivery device 502 can be configured to communicate with a user device 506.

User device 506 can be a computing device as described herein, in some non-limiting embodiments, a smartphone. User device 506 can be programmed or configured to communicate, for example through communication network 508, with a healthcare system 510, for example through a mobile application executable on user device 506.

Communication network 508 may include one or more wired and/or wireless networks. For example, communication network 508 may include a BLUETOOTH connection (e.g., between drug delivery device 502 and user device 506), a cellular network (e.g., a long-term evolution (LTE) network, a third generation (3G) network, a fourth generation (4G) network, a fifth generation (5G) network, a code division multiple access (CDMA) network, etc.), a public land mobile network (PLMN), a UWB network, a local area network (LAN), a low-power wide area network (LPWAN), a wide area network (WAN), a metropolitan area network (MAN), a telephone network (e.g., the public switched telephone network (PSTN) and/or the like), a private network, an ad hoc network, an intranet, the Internet, a fiber optic-based network, a cloud computing network, and/or the like, and/or a combination of some or all of these or other types of networks.

Healthcare system 510 may include a server, a group of servers, and/or other like devices. More than one healthcare system 510 can be provided, for example, a system associated with a device manufacturer, a system associated with a pharmaceutical manufacturer, a system associated with a healthcare provider, a system associated with a government agency, and/or a system associated with a study sponsor, for example a sponsor of a clinical trial.

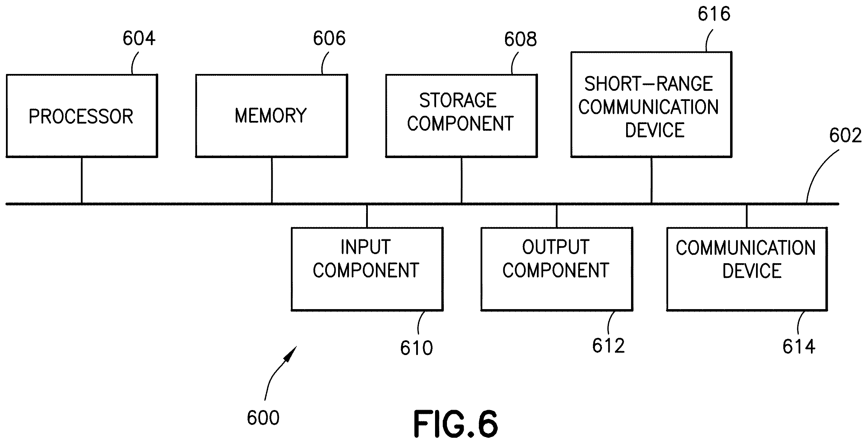

Referring now to FIG. 6, shown is a diagram of example components of an exemplary computing device 600, in an exemplary system, useful for methods described herein. Such a computing device 600 may correspond to a component within the drug delivery device as described herein, a user device as described herein, and/or a healthcare system as described herein. As shown in FIG. 6, a computing device 600 may include bus 602, processor 604, memory 606, storage component 608, input component 610, output component 612, and/or communication interface 614.

Bus 602 may include a component that permits communication among the components of a computing device 600. In some non-limiting embodiments, processor 604 may be implemented in hardware, software, or a combination of hardware and software. For example, processor 604 may include a processor (e.g., a central processing unit (CPU), a graphics processing unit (GPU), an accelerated processing unit (APU), and/or the like), a microprocessor, a digital signal processor (DSP), and/or any processing component (e.g., a field-programmable gate array (FPGA), an application-specific integrated circuit (ASIC), and/or the like) that can be programmed to perform a function. Memory 606 may include random access memory (RAM), read-only memory (ROM), and/or another type of dynamic or static storage memory (e.g., flash memory, magnetic memory, optical memory, and/or the like) that stores information and/or instructions for use by processor 604.

Storage component 608 may store information and/or software related to the operation and use of computing device 600. For example, storage component 608 may include a hard disk (e.g., a magnetic disk, an optical disk, a magneto-optic disk, a solid state disk, and/or the like), a compact disc (CD), a digital versatile disc (DVD), a floppy disk, a cartridge, a magnetic tape, and/or another type of computer-readable medium, along with a corresponding drive.

Input component 610 may include a component that permits computing device 600 to receive information, such as via user input (e.g., a touch screen display, a keyboard, a keypad, a mouse, a button, a switch, a microphone, and/or the like). Additionally or alternatively, input component 610 may include a sensor for sensing information (e.g., a global positioning system (GPS) component, an accelerometer, a gyroscope, an actuator, and/or the like). Output component 612 may include a component that provides output information from a computing device (e.g., a display, a speaker, one or more light-emitting diodes (LEDs), and/or the like).

Communication interface 614 may include a transceiver-like component (e.g., a transceiver, a separate receiver, and transmitter, etc.) that enables device to communicate with other devices, such as via a wired connection, a wireless connection, or a combination of wired and wireless connections. Communication interface 614 may permit computing device 600 to transmit and/or receive information from another device. For example, communication interface 614 may include an Ethernet interface, an optical interface, a coaxial interface, an infrared interface, a radio frequency (RF) interface, a universal serial bus (USB) interface, a Wi-Fi^{®} interface, a cellular network interface, BLUETOOTH interface, UWB interface, and/or the like. In non-limiting embodiments, communication interface 614 operates through one or both of near-field communication and RFID. Suitable communication protocols and methods for securing communications between communication interface 614 and a communication interface of another device, such as a computing device (e.g., desktop computer, laptop computer, smartphone, smart watch, PDA, tablet, etc.,) can include encryption, e.g., using a secure socket layer (SSL) (e.g., by using public/private key pairs as are known in the art). Additional security protocols are disclosed in, for example, U.S. Patent Nos. 9,445,264 and 9,463,325.

A computing device may perform one or more processes described herein. A computing device may perform these processes based on processor 604 executing software instructions stored by a computer-readable medium, such as memory 606 and/or storage component 608, and/or being instructed by a separate computing device. A computer-readable medium (e.g., a non-transitory computer-readable medium) is defined herein as a non-transitory memory device. A non-transitory memory device includes memory space located inside of a single physical storage device or memory space spread across multiple physical storage devices.

Software instructions may be read into memory 606 and/or storage component 608 from another computer-readable medium or from another device via communication interface 614. When executed, software instructions stored in memory 606 and/or storage component 608 may cause processor 604 to perform one or more processes described herein. Additionally or alternatively, hardwired circuitry may be used in place of or in combination with software instructions to perform one or more processes described herein. Thus, embodiments described herein are not limited to any specific combination of hardware circuitry and software.

With reference once again to FIG. 5, in non-limiting embodiments, an exemplary system 500 may include a drug delivery device 502 including short-range communication device 504 as described previously. The drug delivery device 502, through short-range communication device 504, may thus be in communication with a user's computing device 506, such as a smartphone, having its own associated communication interface and processor, as well as memory, storage component, bus, input component, and/or output component.

In non-limiting embodiments, short-range communication device 504 is in one-way communication with a user's computing device 506, e.g., the short-range communication device 504 can only transmit data to the user device 506, and cannot receive data from the user device 506.

As described above, in non-limiting embodiments, upon being placed near drug delivery device 502, user's computing device 506, by virtue of being enabled for NFC, can deliver power to sensor assembly including short-range communication device 504, which can, based on short-range communication device 504 being in electrical communication with capacitive elements, generate and/or record first, second, third, etc. capacitance data which may be transmitted to user's computing device 506, optionally with one or more identifiers (e.g., a device identifier, drug identifier, lot identifier, and/or an identifier associated with short-range communication device 504). In non-limiting embodiments, capacitance data and/or one or more identifiers are encrypted before being transmitted to user device 506. In addition, other sensors can be provided that are in communication with short-range communication device 504 (or any other included communication device, such as an RFID device) and allow for transmission of, for example, GPS data, gyroscope data, temperature data, humidity data, light exposure data, and the like, which may be relevant to any composition that is to be held within drug delivery device 502.

In non-limiting embodiments, user device 506 and/or healthcare system 510, based on software installed thereon, may compare first, second, etc. capacitance data received from drug delivery device 502 to one or more pre-determined capacitance values. Such pre-determined capacitance values may be stored in memory of user device 506 and/or healthcare system 510, and may include predicted or actual capacitance values for various stages of drug delivery. For example, pre-determined capacitance values may include capacitance measured when the syringe is full of a composition (e.g., plastic, glass, a higher density of metal from a compressed needle shield spring, and/or the composition are adjacent capacitive element(s)) (pre-injection), and/or when the syringe has been actuated (e.g., plastic, glass, the rubber stopper, and or a lower density of metal from an expanded spring) are adjacent capacitive element(s)) (post-injection). Results of the comparison(s), as well as received capacitance data and/or one or more identifiers and/or additional data described herein, may be stored on one or both of user device 506 and/or healthcare system 510, and transmitted therebetween via network 508. In non-limiting embodiments, if any capacitance data falls outside of a predetermined range, a message can be generated by or sent to user device 506. In addition, data may be stored by user device 506 and/or healthcare system 510, such that if a particular sensor assembly (e.g., short-range communication device 504) is believed to have not been used previously, but data is detected and/or stored that is indicative of capacitance in a post-use configuration, an alert may be sent to the user device 506 that the drug delivery device 502 may have been previously used or may have been tampered with.

In non-limiting embodiments, healthcare system 510 may be in communication with one or more additional healthcare systems 510. For example, one healthcare system may be maintained by the device manufacturer, and may be in communication with a healthcare system maintained by a study sponsor, pharmaceutical manufacturer, or the like. In non-limiting embodiments, capacitance data (first capacitance data, second capacitance data, etc. and/or any comparisons between the measured capacitance data and predetermined capacitance levels), together with one or more identifiers (e.g., a device identifier and/or an identifier associated with the short-range communication device 504), may be transmitted from device manufacturer system to study sponsor or pharmaceutical manufacturer system, where one or more databases may store data associating the one or more identifiers (e.g., a device identifier and/or an identifier associated with the short-range communication device 504) with a patient identifier, allowing for association of any collected data with the patient.

Although the present disclosure has been described in detail for the purpose of illustration based on what is currently considered to be the most practical and preferred embodiments, it is to be understood that such detail is solely for that purpose and that the present disclosure is not limited to the disclosed embodiments, but, on the contrary, is intended to cover modifications and equivalent arrangements that are within the scope of the appended claims. For example, it is to be understood that the present disclosure contemplates that, to the extent possible, one or more features of any embodiment can be combined with one or more features of any other embodiment.

## Claims

1. A drug delivery device (100) comprising:
a syringe (115) barrel comprising a proximal end, a distal end, and a sidewall arranged between the proximal end and the distal end defining an interior configured to hold a composition;
a plunger rod (118) received at least partially within the interior of the syringe (115), the plunger rod (118) comprising a proximal end, a distal end, and a stopper arranged at the distal end;
a sensor assembly (160), comprising:
a capacitive element (150); and
a short-range communication device (140) in electrical communication with the capacitive element (150), wherein the short-range communication device (140) is capable of measuring capacitance at the capacitive element (150),
**characterized in that** the drug delivery device further comprises:
a displaceable needle shield (110); and
a spring (120) having a compressed configuration and an expanded configuration, the spring (120) configured to displace the needle shield from a first, proximal position in which the displaceable needle shield (110) does not surround a needle (116) of the syringe (115) to a second, distal position in which the displaceable needle shield (110) at least partially surrounds the needle (116) of the syringe (115).
and **in that** the capacitive element (150) is arranged adjacent to the spring (120), such that a change in state of the spring from the compressed configuration to the expanded configuration affects the capacitance of capacitive element (150).

2. The drug delivery device of claim 1, wherein the sensor assembly (160) does not include a power source.

3. The drug delivery device of claim 2, wherein the short-range communication device (140) is an NFC device.

4. The drug delivery device of claim 3, wherein the sensor assembly (160) is powered by an external NFC-enabled device.

5. The drug delivery device of claim 4, wherein the external NFC-enabled device is a mobile device.

6. The drug delivery device of claim 1, wherein the capacitive element (150) comprises a pair of capacitor electrodes.

7. The drug delivery device of claim 1, wherein the capacitive element (150) is arranged at the distal end of the syringe barrel.

8. The drug delivery device of claim 1, wherein the sensor assembly (160) comprises a label arranged on an exterior of the syringe sidewall.

9. The drug delivery device of claim 1, wherein the short-range communication device (140) is a passive RFID chip.

10. The drug delivery device of claim 1, wherein the sensor assembly (160) is arranged on the displaceable needle shield (110).

11. The drug delivery device of claim 10, wherein the capacitive element (150) is arranged on the displaceable needle shield (110) adjacent the spring (120).

12. A system, comprising:
the drug delivery device of claim 1; and
an NFC-enabled mobile device.

13. A method of monitoring drug delivery status, comprising:
providing the drug delivery device of claim 1;
powering, with a mobile device, the sensor assembly to obtain first capacitance data; and
transmitting, with the short-range communication device and to the mobile device, the first capacitance data.

## Patentansprüche

1. Arzneimittelabgabevorrichtung (100), umfassend:
einen Körper einer Spritze (115), der ein proximales Ende, ein distales Ende und eine zwischen dem proximalen Ende und dem distalen Ende angeordnete Seitenwand umfasst, die einen Innenraum definiert, der so konfiguriert ist, dass er eine Zusammensetzung aufnimmt;
eine Kolbenstange (118), die mindestens teilweise im Innenraum der Spritze (115) aufgenommen ist, wobei die Kolbenstange (118) ein proximales Ende, ein distales Ende und einen am distalen Ende angeordneten Anschlag umfasst;
eine Sensorbaugruppe (160), umfassend:
ein kapazitives Element (150); und
eine Nahbereichskommunikationsvorrichtung (140) in elektrischer Kommunikation mit dem kapazitiven Element (150), wobei die Nahbereichskommunikationsvorrichtung (140) in der Lage ist, die Kapazität am kapazitiven Element (150) zu messen,
**dadurch gekennzeichnet, dass** die Arzneimittelabgabevorrichtung ferner Folgendes umfasst:
einen verschiebbaren Nadelschutz (110); und
eine Feder (120), die eine komprimierte Konfiguration und eine expandierte Konfiguration aufweist, wobei die Feder (120) so konfiguriert ist, dass sie den Nadelschutz von einer ersten proximalen Position, in der der verschiebbare Nadelschutz (110) eine Nadel (116) der Spritze (115) nicht umschließt, in eine zweite distale Position verschiebt, in der der verschiebbare Nadelschutz (110) die Nadel (116) der Spritze (115) mindestens teilweise umschließt.
und dass das kapazitive Element (150) neben der Feder (120) so angeordnet ist, dass sich eine Zustandsänderung der Feder von der komprimierten Konfiguration in die expandierte Konfiguration auf die Kapazität des kapazitiven Elements (150) auswirkt.

2. Arzneimittelabgabevorrichtung nach Anspruch 1, wobei die Sensorbaugruppe (160) keine Stromquelle umfasst.

3. Arzneimittelabgabevorrichtung nach Anspruch 2, wobei die Nahbereichskommunikationsvorrichtung (140) eine NFC-Vorrichtung ist.

4. Arzneimittelabgabevorrichtung nach Anspruch 3, wobei die Sensorbaugruppe (160) von einer externen NFC-fähigen Vorrichtung mit Strom versorgt wird.

5. Arzneimittelabgabevorrichtung nach Anspruch 4, wobei die externe NFC-fähige Vorrichtung eine mobile Vorrichtung ist.

6. Arzneimittelabgabevorrichtung nach Anspruch 1, wobei das kapazitive Element (150) ein Paar Kondensatorelektroden umfasst.

7. Arzneimittelabgabevorrichtung nach Anspruch 1, wobei das kapazitive Element (150) am distalen Ende des Spritzenkörpers angeordnet ist.

8. Arzneimittelabgabevorrichtung nach Anspruch 1, wobei die Sensorbaugruppe (160) ein Etikett umfasst, das an einer Außenseite der Spritzenseitenwand angeordnet ist.

9. Arzneimittelabgabevorrichtung nach Anspruch 1, wobei die Nahbereichskommunikationsvorrichtung (140) ein passiver RFID-Chip ist.

10. Arzneimittelabgabevorrichtung nach Anspruch 1, wobei die Sensorbaugruppe (160) auf dem verschiebbaren Nadelschutz (110) angeordnet ist.

11. Arzneimittelabgabevorrichtung nach Anspruch 10, wobei das kapazitive Element (150) auf dem verschiebbaren Nadelschutz (110) neben der Feder (120) angeordnet ist.

12. System, das Folgendes umfasst:
die Arzneimittelabgabevorrichtung nach Anspruch 1; und
eine NFC-fähige mobile Vorrichtung.

13. Verfahren zum Überwachen des Arzneimittelabgabestatus, umfassend:
Bereitstellen der Arzneimittelabgabevorrichtung nach Anspruch 1;
Versorgen der Sensorbaugruppe mit einer mobilen Vorrichtung mit Strom, um erste Kapazitätsdaten zu erhalten; und
Übertragen der ersten Kapazitätsdaten mit der Nahbereichskommunikationsvorrichtung an die mobile Vorrichtung.

## Revendications

1. Dispositif d'administration de médicament (100) comprenant :
un cylindre de seringue (115) comprenant une extrémité proximale, une extrémité distale et une paroi latérale agencée entre l'extrémité proximale et l'extrémité distale définissant une partie intérieure configurée pour contenir une composition ;
une tige de piston (118) reçue au moins partiellement dans la partie intérieure de la seringue (115), la tige de piston (118) comprenant une extrémité proximale, une extrémité distale et un bouchon agencé au niveau de l'extrémité distale ;
un ensemble capteur (160), comprenant :
un élément capacitif (150) ; et
un dispositif de communication à courte portée (140) en communication électrique avec l'élément capacitif (150), dans lequel le dispositif de communication à courte portée (140) est capable de mesurer la capacité au niveau de l'élément capacitif (150),
**caractérisé en ce que** le dispositif d'administration de médicament comprend en outre :
un protège-aiguille déplaçable (110) ; et
un ressort (120) ayant une configuration comprimée et une configuration déployée, le ressort (120) étant configuré pour déplacer le protège-aiguille d'une première position proximale dans laquelle le protège-aiguille déplaçable (110) n'entoure pas l'aiguille (116) de la seringue (115) à une seconde position distale dans laquelle le protège-aiguille déplaçable (110) entoure au moins partiellement l'aiguille (116) de la seringue (115).
et **en ce que** l'élément capacitif (150) est agencé de manière adjacente au ressort (120), de sorte qu'un changement d'état du ressort de la configuration comprimée à la configuration déployée affecte la capacité de l'élément capacitif (150).

2. Dispositif d'administration de médicament selon la revendication 1, dans lequel l'ensemble capteur (160) ne comprend pas de source d'alimentation.

3. Dispositif d'administration de médicament selon la revendication 2, dans lequel le dispositif de communication à courte portée (140) est un dispositif NFC.

4. Dispositif d'administration de médicament selon la revendication 3, dans lequel l'ensemble capteur (160) est alimenté par un dispositif externe compatible NFC.

5. Dispositif d'administration de médicament selon la revendication 4, dans lequel le dispositif externe compatible NFC est un dispositif mobile.

6. Dispositif d'administration de médicament selon la revendication 1, dans lequel l'élément capacitif (150) comprend une paire d'électrodes de condensateur.

7. Dispositif d'administration de médicament selon la revendication 1, dans lequel l'élément capacitif (150) est agencé au niveau de l'extrémité distale du cylindre de seringue.

8. Dispositif d'administration de médicament selon la revendication 1, dans lequel l'ensemble capteur (160) comprend une étiquette agencée sur une partie extérieure de la paroi latérale de seringue.

9. Dispositif d'administration de médicament selon la revendication 1, dans lequel le dispositif de communication à courte portée (140) est une puce RFID passive.

10. Dispositif d'administration de médicament selon la revendication 1, dans lequel l'ensemble capteur (160) est agencé sur le protège-aiguille déplaçable (110).

11. Dispositif d'administration de médicament selon la revendication 10, dans lequel l'élément capacitif (150) est agencé sur le protège-aiguille déplaçable (110) de manière adjacente au ressort (120).

12. Système, comprenant :
le dispositif d'administration de médicament selon la revendication 1 ; et
un dispositif mobile compatible NFC.

13. Procédé de surveillance de l'état d'administration de médicament, comprenant :
la fourniture du dispositif d'administration de médicament selon la revendication 1 ;
l'alimentation, au moyen d'un dispositif mobile, de l'ensemble capteur afin d'obtenir des premières données de capacité ; et
la transmission, au moyen du dispositif de communication à courte portée et vers le dispositif mobile, des premières données de capacité.
